(19) Europäisches Patentamt
European Patent Office
Office européen des brevets

(11) Publication number: **0 200 132**
**A2**

# (12) EUROPEAN PATENT APPLICATION

(21) Application number: **86105470.8**

(51) Int. Cl.4: **C07D 313/14** , A61K 31/335

(22) Date of filing: **21.04.86**

(30) Priority: **23.04.85 US 726432**

(43) Date of publication of application:
**10.12.86 Bulletin 86/45**

(84) Designated Contracting States:
**AT BE CH DE FR GB IT LI LU NL SE**

(71) Applicant: **HOECHST-ROUSSEL PHARMACEUTICALS INCORPORATED**
**Route 202-206 North**
**Somerville New Jersey 08876(US)**

(72) Inventor: **Ong, Helen Hu**
**15 Bunker Hill Road**
**Whippany New Jersey 07981(US)**

(74) Representative: **Becker, Heinrich Karl Engelbert, Dr. et al**
**HOECHST AKTIENGESELLSCHAFT Central Patent Department P.O. Box 80 03 20**
**D-6230 Frankfurt am Main 80(DE)**

## (54) (Aminoalkylthio)hydroxydibenzoxepins, a process for their preparation and their use as medicaments.

(57) The present invention relates to novel (aminoalkylthio)hydroxydibenz[b,f]oxepins and to a process for their preparation. The compounds show antidepressant and analgesic activity and can, therefore, be used as medicaments.

EP 0 200 132 A2

# (Aminoalkylthio)hydroxydibenzoxepins, a process for their preparation and their use as medicaments

The present invention relates to novel (aminoalkylthio)hydroxydibenzoxepins. More particularly, the present invention relates to (aminoalkylthio)hydroxydibenzoxepins of the formula Ia

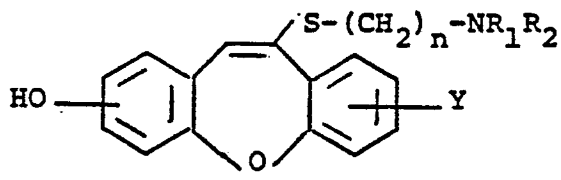

1

wherein $R_1$ is hydrogen, loweralkyl of 1 to 6 carbon atoms or a group of the formula

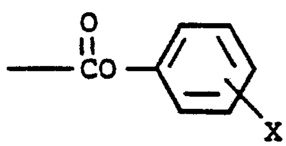

wherein X is hydrogen, halogen, loweralkyl of 1 to 6 carbon atoms, loweralkoxy of 1 to 6 carbon atoms, trifluoromethyl or nitro; $R_2$ is loweralkyl of 1 to 6 carbon atoms; Y is hydrogen, halogen, hydroxy, trifluoromethyl, loweralkyl of 1 to 6 carbon atoms, loweralkoxy of 1 to 6 carbon atoms, loweralkylthio of 1 to 6 carbon atoms, loweralkylsulfinyl of 1 to 6 carbon atoms, loweralkylsulfonyl of 1 to 6 carbon atoms, amino or nitro; and n is 2, 3, or 4, the optical antipodes thereof, or a pharmaceutically acceptable salt thereof, which are useful for treating depression and alleviating pain, alone or in combination with inert adjuvants.

Preferred (aminoalkylthio)hydroxydibenzoxepins of the present invention are those wherein $R_1$ is hydrogen; Y is halogen; and n is 2.

Included within the (aminoalkylthio)hydroxydibenzoxepins of the present invention are (phenoxycarbonylaminoalkylthio)hydroxydibenzoxepins of formula 1 wherein $R_1$ is a group of the formula

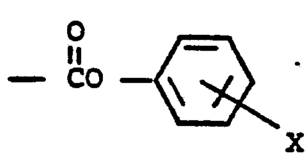

wherein X is as above, as intermediates for the preparation of compounds of the present invention wherein $R_1$ is hydrogen. Preferred (phenoxycarbonylaminoalkylthio)hydroxydibenzoxepins of the present invention are those wherein X is hydrogen, Y is halogen and n is 2.

As used through the specification and appended claims, the term "alkyl" refers to a straight or branched chain hydrocarbon radical containing no unsaturation and having 1 to 10 carbon atoms such as methyl, ethyl, 1-propyl, 2-propyl, 1-butyl, 1-pentyl, 2-pentyl, 3-hexyl, 4-heptyl, 2-octyl, 3-nonyl, 4-decyl and the like; the term "alkanol" refers to a compound formed by a combination of an alkyl group and a hydroxy radical. Examples of alkanols are methanol, ethanol, 1-and 2-propanol, 1,2-dimethylethanol, hexanol, octanol, decanol and the like. The term "alkoxy" refers to a group formed by removal of a hydrogen function from the hydroxy group of an alkanol. Examples of alkoxy groups are methoxy, ethoxy, 1-and 2-propoxy, 1,2-dimethylethoxy, hexoxy, octoxy, decoxy and the like. The term "halogen" refers to a member of the family, fluoro, chloro, bromo, and iodo. The term "lower" as applied to any of the aforementioned groups refers to a group having a carbon skeleton containing up to and including 6 carbon atoms.

The compounds of the present invention which lack an element of symmetry exist as optical antipodes and as the racemic forms thereof. The optical antipode may be prepared from the corresponding racemic forms by standard optical resolution techniques, involving, for example, the separation of diasteromeric salts of those instant compounds characterized by the presence of a basic amino group and an optically active acid, or by the synthesis from optically active precursors.

The present invention comprehends all optical isomers and racemic forms thereof of the compounds disclosed and claimed herein. The formulas of the compounds shown herein are intended to encompass all optical isomers of the compounds so depicted.

The novel (aminoalkylthio)-hydroxydibenzoxepins of the present invention wherein $R_1$ is hydrogen are synthesized by dealkylation of an alkoxy(aminoalkylthio)dibenzoxepin of formula 2

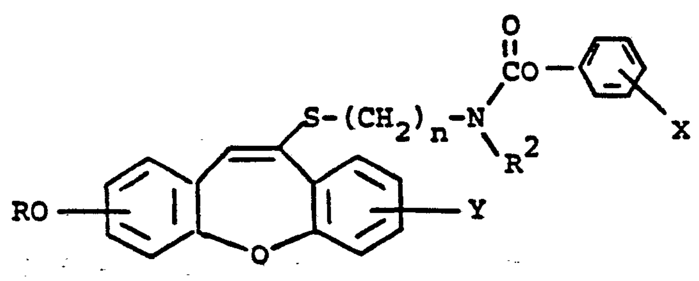

2

wherein R is loweralkyl, $R_2$ is loweralkyl, and X, Y and n are as hereinbeforedescribed, the preparation of which is disclosed in European Patent No. 000 2508, to afford an (aminoalkylthio)-hydroxybenzoxepin of formula 3

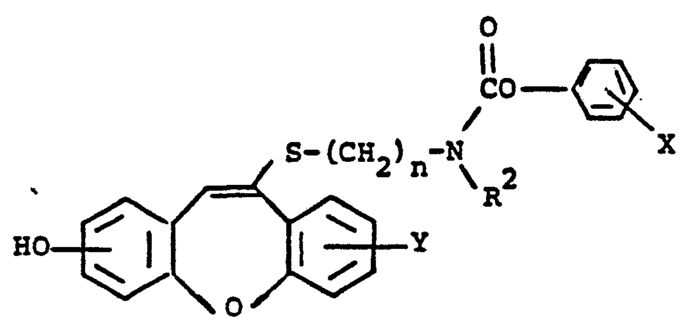

3

wherein $R_2$, X, Y, and n are as above, followed by hydrolysis of the phenoxycarbonyl group of 3 to provide a compound of formula 4

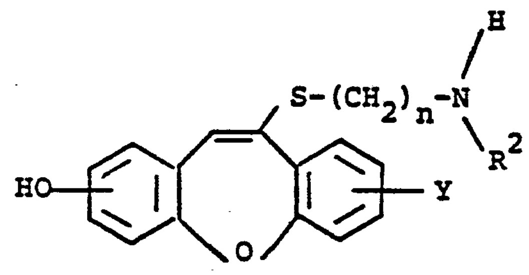

4

wherein $R_2$, Y and n are as above.

The dealkylation is conveniently performed by contacting an alkoxy compound of formula 2 with a boron trihalide of formula 5

$$BHal_3$$

5

wherein Hal is halogen in a halocarbon solvent. Among boron halides there may be mentioned boron triiodide, boron tribromide and boron trichloride. Boron tribromide is preferred. Among halocarbon solvents there may be mentioned dichloromethane, trichloromethane, 1,1-dichloroethane, 1,2-dichloroethane, and the like. Dichloromethane is preferred. While the dealkylation temperature is not narrowly critical, it is preferred to conduct the reaction at a temperature within the range of about 0° to 50°C, the most preferred temperature being about 25°C.

The hydrolysis of an N-(phenoxycarbonylalkylthio)dibenzoxepin is accomplished by treating 3 with an alkali metal hydroxide such as lithium hydroxide, sodium hydroxide or potassium hydroxide in an aqueous alkanol such as methanol, ethanol, 1-or 2-propanol, 2,2-dimethylethanol and the like, or an aqueous alkandiol such as 1,2-dihydroxyethane, 1,2-dihydroxypropane, 1,3-dihydroxypropane and the like. The conversion proceeds smoothly at moderate temperatures. However, when an alkandiol is utilized as the solvent, elevated temperatures within the range of about 125° to about 175°C are employed to promote the reaction. Potassium hydroxide, aqueous 1,2-dihydroxyethane and the reaction temperatures of about 150° to about 165°C are preferred.

The novel (aminoalkylthio)-hydroxydibenzoxepins of the present invention wherein $R_1$ is loweralkyl may be prepared, for example, by dealkylation of an alkoxy(aminoalkylthio)-dibenzoxepin of formula 6

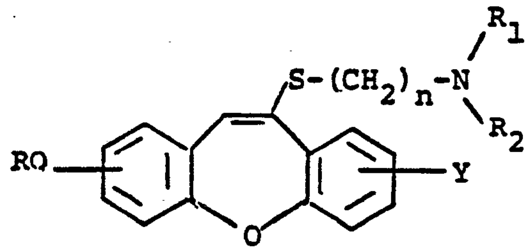

6

wherein R, $R_1$, and $R_2$ are loweralkyl, and Y and n are as hereinbeforedescribed, the synthesis of which is described in U.S. Patent Application Docket No. AHC 80 CIP-Cont-CIP-CIP, filed even date herewith, by the process for the conversion of a compound of formula 2 to a compound of formula 3 to provide an (aminoalkylthio)-hydroxydibenzoxepin of formula 7

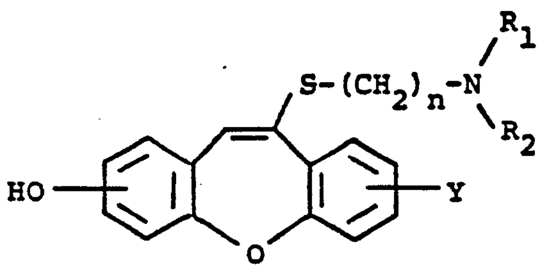

7

wherein $R_1$ and $R_2$ are loweralkyl and Y and n are as hereinbeforedescribed.

The (aminoalkylthio)hydroxydibenzoxepins of the present invention are useful in the treatment of depression in mammals, as demonstrated by their ability to inhibit tetrabenazine-induced depression in mice [International Journal of Neuropharmacology 8, 73 (1969)], a standard assay for the determination of antidepressant properties. Thus, for instance, the intraperitoneal dose at which 2-fluoro-8-hydroxy-11-[β-(methylamino)ethylthio]dibenz[b,f]-oxepin effects a 50% inhibition of tetrabenazine-induced ptosis ($ED_{50}$) in mice is 3.3 mg/kg of body weight, and the intraperitoneal dose at which 2-fluoro-7-hydroxy-11-[β-(methylamino)ethylthio]-dibenz[b,f]oxepin effects an 89% inhibition of tetrabenazine-induced ptosis in mice is 20 mg/kg of body weight. Amitriptyline, a standard antidepressant, exhibits an $ED_{50}$ of 1.5, intraperitoneally, in this assay.

Antidepressant response is achieved when the present (aminoalkylthio)hydroxydibenzoxepins are administered to a subject requiring such treatment as an effective oral, parenteral or intravenous dose of from 0.01 to 100 mg/kg of body weight per day. A particularly effective amount is about 25 mg/kg of body weight per day. It is to be understood, however, that for any particular subject, specific dosage regimens should be adjusted according to the individual need and the professional judgment of the person administering or supervising the administration of the aforesaid compound. It is to be further understood that the dosages set forth herein are exemplary only and that they do not, to any extent, limit the scope or practice of the invention.

The (aminoalkylthio)hydroxydibenzoxepins of the present invention are also useful as analgetic agents due to their ability to alleviate pain in mammals. The analgetic utility of compounds of this invention is demonstrated in the phenyl-p-quinone writhing assay in mice, a standard assay for analgesia [Proc. Soc. Exptl. Biol. Med., 95 729 - (1957)].Thus, for example, the subcutaneous dose effecting a 69% inhibition of writhing in mice in this assay is 20 mg/kg of body weight for 2-fluoro-8-hydroxy-11-[β-(methylamino)ethylthio]dibenz[b,f]-oxepin and the subcutaneous dose effecting a 42% inhibition of writhing is 10 mg/kg of body weight for 2-fluoro-7-hydroxy-11-[β-(methylamino)ethylthio]-

dibenz[b, f]-oxepin. Propoxyphene, a standard analgetic, exhibits a calculated 50% inhibition of writhing at 3.9 mg/kg of body weight by subcutaneous administration in this assay.

Analgetic production is achieved when the present (aminoalkylthio)hydroxydibenzoxepins are administered to a subject requiring such treatment as an effective oral, parenteral or intravenous dose of from 0.01 to 100 mg/kg of body weight per day. A particularly effective amount is about 25 mg/kg of body weight per day. It is to be understood, however, that for any particular subject, specific dosage regimens should be adjusted according to the individual need and the professional judgement of the person administering of the administering of the aforesaid compound. It is to be further understood that the dosages set forth herein are exemplary only and that they do not, to any extent, limit the scope or practice of the invention.

Additional (aminoalkylthio)-hydroxydibenzoxepins of the invention include:

a. 8-hydroxy-2-trifluoromethyl-11-[γ-(dimethylamino) propylthio]dibenz[b,f]oxepin;

b. 2-fluoro-8-hydroxy-11-{β-[N-methyl-N-(4-chloro phenoxycarbonyl)amino]-ethylthio}dibenz[b,f]oxepin;

c. 2-fluoro-8-hydroxy-11-{γ-N-methyl-N-(3-methyl phenoxycarbonyl)amino]-propyl}dibenz[b,f)oxepin;

d. 2-fluoro-8-hydroxy-11-{δ-N-methyl-N-(2-methoxy phenoxycarbony)amono]-butyl}dibenz[b,f]oxepin;

e. 2-fluoro-7-hydroxy-11-{β-]N-methyl-N-(4-trifluoro methylphenoxycarbonyl)amino]-ethylthio}dibenz[b,f]oxepin;

f. 2-fluoro-7-hydroxy-11-{γ-[N-methyl-N-(3-nitro methylphenoxycarbonyl)amino]-propyl}dibenz[b,f)oxepin;

g. 3-ethyl-8-hydroxy-11-]β-(methylamino)-ethylthio] dibenz[b,f]oxepin;

h. 4-methoxy-7-hydroxy-11-[γ-(diethylamino)propylthio) dibenz[b,f]oxepin;

i. 6-hydroxy-2-methylthio-11-[δ-(ethylamino)-butylthio) dibenz[b,f]oxepin;

j. 3-ethylsulfonyl-9-hydroxy-11-[β-(dimethylamino) ethylthio]dibenz[b,f]oxepin;

k. 4-ethylsulfinyl-7-hydroxy-11-[γ-(dimethylamino) propylthio]dibenz[b,f]oxepin;

l. 2-amino-8-hydroxy-11-[β(ethylamino)-ethylthio] dibenz[b,f]oxepin;

m. 7-hydroxy-2-nitro-11-[β(methylamino)-ethylthio] dibenz[b,f]oxepin;

n. 2,7-dihydroxy-11-[β-(dimethylamino)-ethylthio) [b,f]oxepin.

Effective amounts of the compounds of the present invention may be administered to a subject by any one of various methods, for example, orally as in capsules or tablets, parenterally in the form of sterile solutions or suspensions, and in some cases intravenously in the form of sterile solutions. The -(aminoalkylthio)hydroxydibenzoxepins of the present invention, while effective themselves, may be formulated and administered in the form of their pharmaceutically acceptable addition salts for purposes of stability, convenience, crystallization, increased solubility and the like.

Preferred pharmaceutically acceptable addition salts include salts of mineral acids, for example, hydrochloric acid, sulfuric acid, nitric acid and the like, salts of monobasic carboxylic acids such as, for example, acetic acid, propionic acid and the like, salts of dibasic carboxylic acids such as, for example, maleic acid, fumaric acid and the like, and salts of tribasic carboxylic acids such as, for example, carboxysuccinic acid, citric acid and the like.

Effective quantities of the compounds of the invention may be administered orally, for example, with an inert diluent or with an edible carrier. They may be enclosed in gelatin capsules or compressed into tablets. For the purpose of oral therapeutic administration, the aforesaid compounds may be incorporated with excipients and used in the form of tablets, troches, capsules, elixirs, suspensions, syrups, wafers, chewing gums and the like. These preparations should contain at least 0.5% of active compound, but may be varied depending upon the particular form and may conveniently be between 4% to about 70% of the weight of the unit. The amount of active compound in such composition is such that a suitable dosage will be obtained. Preferred compositions and preparations according to the present invention are prepared so that an oral dosage unit form contains between 1.0-300 milligrams of the active compound.

The tablets, pills, capsules, troches and the like may also contain the following ingredients: a binder such as microcrystalline cellulose, gum tragancanth or gelatin; an excipient such as starch or lactose, a

disintegrating agent such as alginic acid, corn starch and the like; a lubricant such as magnesium stearate; a glidant such as colloidal silicon dioxide; and a sweetening agent such as sucrose or saccharin or a flavoring agent such as peppermint, methyl salicylate, or orange flavoring may be added. When the dosage unit form is a capsule, it may contain, in addition to materials of the above type, a liquid carrier such as a fatty oil. Other dosage unit forms may contain other various materials which modify the physical form of the dosage unit, for example, as coating. Thus, tablets or pills may be coated with sugar, shellac, or other enteric coating agents. A syrup may contain, in addition to the active compounds, sucrose as a sweetening agent and certain preservatives, dyes and colorings and flavors. Materials used in preparing these various compositions should be pharmaceutically pure and non-toxic in the amounts used.

For the purposes of parenteral therapeutic administration, the active compounds of the invention may be incorporated into a solution or suspension. These preparations should contain at least 0.1% of active compound, but may be varied between 0.5 and about 50% of the weight thereof. The amount of active compounds in such compositions is such that a suitable dosage will be obtained. Preferred compositions and preparations according to the present invention are prepared so that a parenteral dosage unit contains between 0.5 to 100 milligrams of active compound.

The solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

The following examples are for illustrative purposes only and are not to be construed as limiting the invention.

EXAMPLE 1

2-Fluoro-8-hydroxy-11-[$\beta$-(N-methyl-N-phenoxycarbonylamino)-ethylthio]dibenz[b,f]oxepin

A solution of 2.1 g of 2-fluoro-8-methoxy-11-[$\beta$-(N-methyl-N-phenoxycarbonyl)ethylthio]dibenz[b,f]-oxepin in 10 ml of dichloromethane was cooled while 6 ml of a stock solution of 1M boron tribromide in dichloromethane was added dropwise. Stirring was continued at room temperature for 16 hrs before the reaction mixture was quenched with 100 ml of water. The organics were extracted into ether, washed and dried over anhydrous magnesium sulfate. Removal of the solvent under reduced pressure left an oil which was passed through a column of silica gel packed in ether and eluted with ether to yield 1.65 g (58%) of product, m.p. 109°C, after trituration with hexane.

**ANALYSIS:**

| | | | |
|---|---|---|---|
| Calculated for $C_{24}H_{20}FNO_4S$: | 65.89%C | 4.61%H | 3.20%N |
| Found: | 66.29%C | 4.81%H | 2.99%N |

EXAMPLE 2

2-Fluoro-8-hydroxy-11-[$\beta$-(methylamino)ethylthio)-dibenz -[b,f]oxepin

A mixture of 1.5 g of 2-fluoro-8-hydroxy-11-[$\beta$-(N-methyl-N-phenoxycarbonylamino)ethylthio]-dibenz[b,f] oxepin, 3.0 g of 85% potassium hydroxide in 10 ml of ethylene glycol was stirred at 165°C for 1 hr. The cooled solution was diluted with water and made acidic (pH=2) with conc hydrochloric acid. The mixture was extracted with ether, the aqueous solution was basified with potassium carbonate solution and extracted exhaustively with chloroform. The combined organic extracts were washed with water, dried over anhydrous magnesium sulfate and the solvent was removed in vacuo. Recrystallized from acetone-hexane gave 750 mg (69.5%) of product, mp 159-160°C.

**ANALYSIS:**

| | | | |
|---|---|---|---|
| Calculated for $C_{17}H_{16}FNO_2S$: | 64.33%C | 5.08%H | 4.41%N |
| Found: | 64.25%C | 5.25%H | 4.40%N |

EXAMPLE 3

2-Fluoro-7-hydroxy-11-[β-(methylamino)ethylthio]-dibenz-[b,f]oxepin

To a solution of 1.0 g of 2-fluoro-7-methoxy-11-[β-(N-methyl-N-phenoxycarbonyl)ethylthio]dibenz-[b,f] oxepin in 5 ml of dichloromethane was added dropwise 4 ml of 1M boron tribromide in dichloromethane. After stirring at room temperature for 15 hrs, the mixture was equilibrated with 20 ml of ether and 10 ml of 2N hydrochloric acid. Stirring was continued for 1 hr, and layers were separated. The organic phase was washed with water, dried over anhydrous magnesium sulfate and concentrated to give 2-fluoro-7-hydroxy-11-[β-(N-methyl-N-phenoxycarbonylamino)ethylthio]dibenz[b,f] oxepin as an oil.

The carbamate (0.5 g) was dissolved in 3 ml of ethylene glycol and heated with 1.0 g of potassium hydroxide in 2 ml of water under nitrogen at 150°C for 3 hrs. The cooled mixture was diluted with 100 ml of water and acidified to pH=2 with conc hydrochloric acid. The neutral and acidic components were removed by ether extraction, and the aqueous solution was saturated with solid potassium carbonate. The mixture was extracted with a large excess of chloroform, washed and dried. Evaporation of solvent gave a 185 mg (27%) of product. An analytical sample, prepared by recrystallization from acetone-hexane, had m.p. 181-182°C.

**ANALYSIS:**

| | | | |
|---|---|---|---|
| Calculated for $C_{17}H_{16}FNO_2S$: | 64.33%C | 5.08%H | 4.41%N |
| Found: | 63.91%C | 5.10%H | 4.54%N |

**Claims**

1. A compound of the formula I

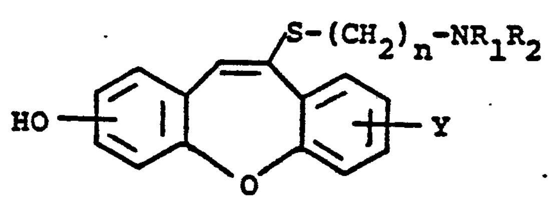

I

wherein $R_1$ is hydrogen, loweralkyl of 1 to 6 carbon atoms or a group of the formula

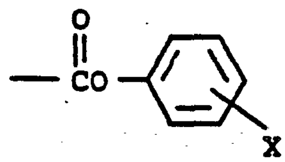

wherein X is hydrogen, halogen, loweralkyl of 1 to 6 carbon atoms, loweralkoxy of 1 to 6 carbon atoms, trifluoromethyl or nitro; $R_2$ is loweralkyl of 1 to 6 carbon atoms; Y is hydrogen, halogen, hydroxy, trifluoromethyl, loweralkyl of 1 to 6 carbon atoms, loweralkoxy of 1 to 6 carbon atoms, loweralkylthio of 1 to 6 carbon atoms, loweralkylsulfinyl of 1 to 6 carbon atoms, loweralkylsulfonyl of 1 to 6 carbon atoms, amino or nitro; and n is 2, 3 or 4, the optical antipodes thereof or a pharmaceutically acceptable salt thereof.

2. A compound according to claim 1 where $R_1$ is hydrogen; Y is halogen; and n is 2.

3. A compound according to claim 1 wherein $R_1$ is a group of the formula

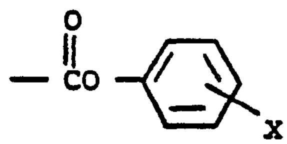

wherein X is hydrogen; Y is halogen; and n is 2.

4. The compound according to claim 2 which is 2-fluoro-7-hydroxy-11-[β-(methylamino)ethylthio]-dibenz[b,f]oxepin.

5. The compound according to claim 2 which is 2-fluoro-8-hydroxy-11-[β-(methylamino)ethylthio]-dibenz[b,f]oxepin.

6. The compound according to claim 3 which is 2-fluoro-7-hydroxy-11-[β-(N-methyl-N-phneoxycarbonylamino)ethylthio]dibenz[b,f]oxepin.

7. The compound according to claim 3 which is 2-fluoro-8-hydroxy-11-[β-(N-methyl-N-phenoxycarbonylamino)ethylthio]dibenz[b,f]oxepin.

8. A process for the preparation of a compound of the formula I

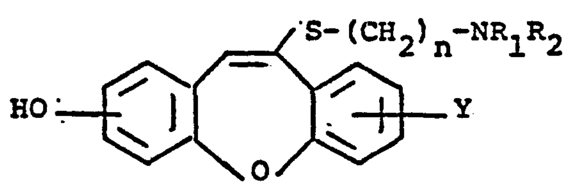

I

wherein $R_1$ is hydrogen, loweralkyl of 1 to 6 carbon atoms or a group of the formula

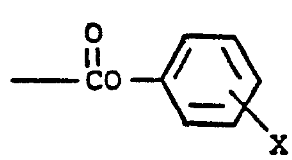

wherein X is hydrogen, halogen, loweralkyl of 1 to 6 carbon atoms, loweralkoxy of 1 to 6 carbon atoms, trifluoromethyl or nitro; $R_2$ is loweralkyl of 1 to 6 carbon atoms; Y is hydrogen, halogen, hydroxy, trifluoromethyl, loweralkyl of 1 to 6 carbon atoms, loweralkoxy of 1 to 6 carbon atoms, loweralkylthio of 1 to 6 carbon atoms, loweralkylsulfinyl of 1 to 6 carbon atoms, loweralkylsulfonyl of 1 to 6 carbon atoms, amino or nitro; and n is 2, 3, or 4, the optical antipodes thereof, or a pharmaceutically acceptable salt thereof, which comprises dealkylating a compound of the formula II

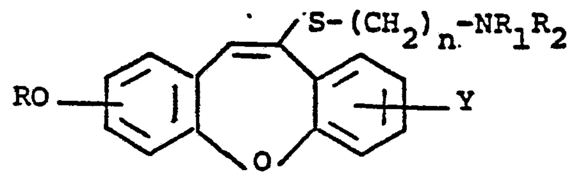

II

wherein Y is as defined above, R is loweralkyl of 1 to 6 carbon atoms, $R_1$ is loweralkyl of 1 to 6 cabon atoms or a group of the formula

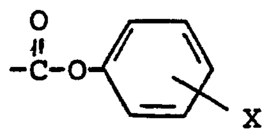

where X is as defined above, and $R_2$ is loweralkyl of 1 to 6 carbon atoms, to provide a compound of the formula I

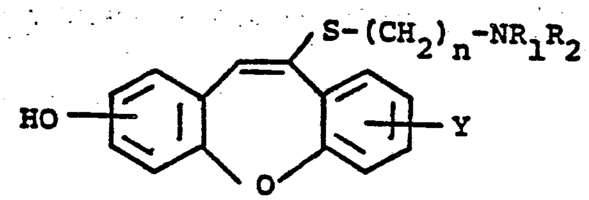

I

wherein Y, $R_1$ and $R_2$ are as defined above, and optionally hydrolyzing a compound of the formula I wherein $R_1$ is a group of the formula

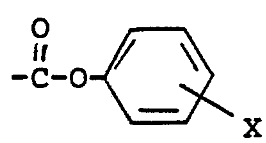

and $R_2$ and Y are as defined above, to provide a compound of the formula I wherein $R_1$ is hydrogen and $R_2$ and Y are as defined above, and optionally preparing a pharmacologically acceptable salt thereof.

9. The process as defined in claim 8 wherein the dealkylation of the compound of the formula II is performed by treating the compound of the formula II with a boron trihalide of the formula $BHal_3$ in a halocarbon solvent.

10. The process as defined in claim 9 wherein boron tribromide in a halogenated alkane as a

solvent is used.

11. A pharmaceutical composition which comprises as active ingredient a compound as defined in claim 1 in association with a pharmaceutically acceptable carrier.

12. Use of a compound as defined in claim 1 for the preparation of a medicament having antidepressant and/or analgesic activity.

Claims for contracting state : AT

1. A process for the preparation of a compound of the formula I

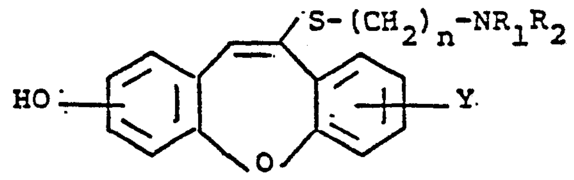

I

wherein $R_1$ is hydrogen, loweralkyl of 1 to 6 carbon atoms or a group of the formula

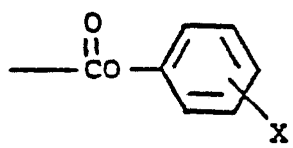

wherein X is hydrogen, halogen, loweralkyl of 1 to 6 carbon atoms, loweralkoxy of 1 to 6 carbon atoms, trifluoromethyl or nitro; $R_2$ is loweralkyl of 1 to 6 carbon atoms; Y is hydrogen, halogen, hydroxy, trifluoromethyl, loweralkyl of 1 to 6 carbon atoms, loweralkoxy of 1 to 6 carbon atoms, loweralkylthio of 1 to 6 carbon atoms, loweralkylsulfinyl of 1 to 6 carbon atoms, loweralkylsulfonyl of 1 to 6 carbon atoms, amino or nitro; and n is 2, 3, or 4, the optical antipodes thereof, or a pharmaceutically acceptable salt thereof, which comprises dealkylating a compound of the formula II

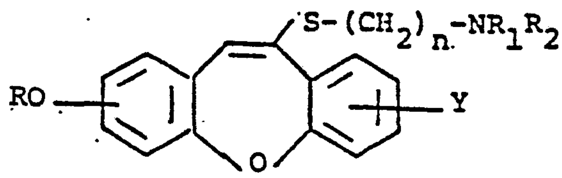

II

wherein Y is as defined above, R is loweralkyl of 1 to 6 carbon atoms, $R_1$ is loweralkyl of 1 to 6 cabon atoms or a group of the formula

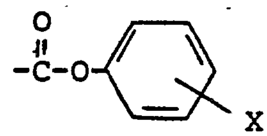

where X is as defined above, and $R_2$ is loweralkyl of 1 to 6 carbon atoms, to provide a compound of the formula I

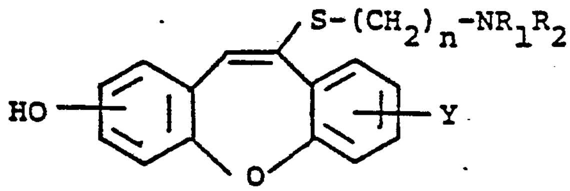

I

wherein Y, $R_1$ and $R_2$ are as defined above, and optionally hydrolyzing a compound of the formula I wherein $R_1$ is a group of the formula

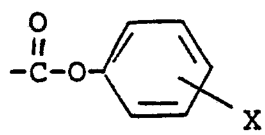

and $R_2$ and Y are as defined above, to provide a compound of the formula I wherein $R_1$ is hydrogen and $R_2$ and Y are as defined above, and optionally preparing a pharmacologically acceptable salt thereof.

2. The process as defined in claim 8 wherein the dealkylation of the compound of the formula II is performed by treating the compound of the formula II with a boron trihalide of the formula $BHal_3$ in a halocarbon solvent.

3. The process as defined in claim 9 wherein boron tribromide in a halogenerated alkane as a solvent is used.

4. The process as defined in claims 1 -3, wherein a compound of the formula I is prepared, where $R_1$ is hydrogen, Y is halogen and n is 2.

5. The process as defined in claims 1 -3, wherein a compound of the formula I is prepared where Y is halogen, n is 2 and $R_1$ is a group of the formula

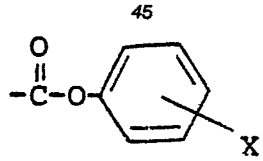

where X is hydrogen.

6. The process as defined in claim 4 wherein the compound 2-fluoro-7-hydroxy-11-[$\beta$-(methylamino)-ethylthio]dibenz[b, f]-oxepin is prepared.

7. The process as defined in claim 4 wherein the compound 2-fluoro-8-hydroxy-11-[$\beta$-(methylamino)-ethylthio]dibenz[b,f]oxepin is prepared.

8. The process as defined in claim 5 wherein the

compound 2-fluoro-7-hydroxy-11-[$\beta$-(N-methyl-N-phenoxycarbonylamino)ethylthio]-dibenz[b,f]oxepin is prepared.

9. The process as defined in claim 5 wherein the compound 2-fluoro-8-hydroxy-11-[$\beta$-(N-methyl-N-phenoxycarbonylamino)ethylthio]-dibenz[b,f]oxepin is prepared.